# EUROPEAN PATENT APPLICATION

(11) **EP 4 696 302 A1**
(43) Date of publication of application: **18.02.2026**
(21) Application number: 25192755.4
(22) Date of filing: 30.07.2025
(51) Int. Cl.: A61K 9/02

(54) **SYSTEM AND METHOD FOR LUBRICANT DELIVERY**

(30) Priority: 13.08.2024 US 202418802871
(71) Applicant: Growth Armor, LLC, Wilmington, DE 19801 (US)
(72) Inventor: Bain, Michael A., Costa Mesa, California, 92627 (US)
(74) Representative: Cohausz & Florack

(57) **Abstract**

Disclosed are suppositories and methods of use thereof for lubricating an internal surface of a body cavity. A suppository may be composed of a shell defining an internal volume and a lubricant at least partially occupying the internal volume. The shell may be prone to degrade in that a rate of degradation thereof may increase when the suppository is in a certain condition, such as being exposed to an external environment or being positioned inside the body cavity. Once sufficient degradation of the shell has occurred while the suppository is inside the body cavity, the lubricant may be released and applied onto an internal surface of the body cavity. Medical procedures or pleasurable activities related to the body cavity may then be performed which rely upon or are aided in part by the lubrication of the body cavity's internal surface.

## Description

### CROSS-REFERENCE TO RELATED APPLICATIONS

Not Applicable

### STATEMENT RE: FEDERALLY SPONSORED RESEARCH/DEVELOPMENT

Not Applicable

### BACKGROUND

### 1. Technical field

The present application relates to lubrication of body cavities like a human anus or vagina. More specifically, the present application relates to suppositories having a degradable shell which releases lubricant stored therein following insertion of the suppository into a body cavity.

### 2. Related Art

The lubrication of internal surfaces of body cavities is a well-versed practice when performing medical procedures or a pleasure-based activity. Surgical or treatment-based procedures for the vaginal and anal regions may depend on lubricated surfaces so that instruments can be inserted into and operate upon the body cavity while minimizing harm to the patient that could stem from abrasion or irritation. Along similar lines, lubrication of body cavities may be necessary in order to perform pleasurable activities like sexual intercourse or using sex toys with those body cavities. Devices and methods pertaining to medical and pleasure-based activities are discloses in Applicant's previously filed patent applications, which include US Patent Publication No. 2023/0201071 entitled "ENHANCED PROPHYLACTICS FOR VACUUM THERAPY", US Patent Publication No. 2023/0293863 entitled "IMPROVED DILATION DEVICE INCORPORATING INFLATABLE BALOON", US Patent Publication No. 2023/0414448 entitled "DILATION DEVICE INCORPORATING ENHANCED FEATURES", US Patent Publication No. 2024/0082552 entitled "RESILIENT ADHESIVE SHEATH FOR USE WITH DILATION DEVICE", US Patent Application No. 18/186,820 entitled "SYSTEMS AND METHODS FOR IMPROVED ENHANCED DILATION DEVICES FOR TARGETED DILATION DIAGNOSTSICS AND THERAPIES", US Patent Application No. 18/297,428 entitled "SYSTEM AND METHOD FOR TREATING FECAL IMPACTION", and US Patent Application No. 18/412,889 entitled "METHOD AND PRODUCTS FOR ENHANCING BLOOD FLOW TO AN ANATOMICAL REGION", the disclosures of each are hereby wholly incorporated by reference.

While biocompatible lubricants have been developed and used to promote such activities, an issue remains as to the delivery of these lubricants. Given that lubricants are conventionally applied from a position external to the body cavity, lubricants are often not applied upon a full region of interest. In particular, deeper regions of a body cavity may not properly be lubricated. It can be seen that new systems and methods are desired in the art which provide a simple lubrication delivery method for a body cavity that are capable of lubricating hard-to-reach regions of the body cavity.

### BRIEF SUMMARY

To solve these and other problems, suppositories and methods of use thereof are disclosed for lubricating an internal surface of a body cavity. A suppository may be composed of a shell defining an internal volume and a lubricant occupying at least a portion of that internal volume. The shell may be prone to degradation such that a rate of degradation thereof increases after exposure to a certain environment, like inside a body cavity. When a sufficient degree of degradation has occurred, the lubricant may be released and coat nearby surfaces. By virtue of the suppository being capable of insertion into a body cavity at a certain depth, the lubricant, when released, may extensively cover areas of the body cavity which alternative lubrication products and methods may be ineffective at reaching.

A suppository may be primarily intended to lubricate an internal surface of a body cavity. In this respect, the suppository could consist essentially of a shell and a lubricant occupying at least a portion of an internal volume defined by the shell. If the lubricant does not fully occupy the internal volume, a void may exist, which may or may not be occupied by a gas like air or inert nitrogen gas. The lubricant could comprise at least 20% by weight, at least 40% by weight, at least 60% by weight, or at least 80% by weight of the total weight of the suppository. The lubricant could be a water-based lubricant, a silicone-based lubricant, or an oil-based lubricant.

The shell may have a rate of degradation which could be smaller when the suppository is in a first condition (e.g., a minimal or zero rate of degradation) when compared to a second condition (where an appreciable rate of degradation can occur). For example, the suppository may be sealed in a packaged which shields the suppository from an external environment; when the package is opened, the suppository may be exposed to the external environment, bringing with it a condition, like an increased moisture content, which increases the rate of degradation of the shell. In another example, the suppository's rate of degradation can increase after insertion into a body cavity, thanks in part to the suppository absorbing heat from the body cavity and/or the body cavity applying pressure to the suppository. The shell could be composed of agarose, fibrin, fibrinogen, collagen, alginate, chitosan, methylcellulose, polyglycolide, poly(lactide-co-glycolide) acid (or PLGA), polyethylene glycol, or combinations thereof.

The shape of the suppository may be generally smooth so as to promote ease of insertion into a body cavity and minimize irritation and harm that rough or edged suppository may impose on a user. In this respect, the suppository could have a distal end, which may have a frustoconical geometry which terminates in a smooth tip, and a proximal end, which could have a flat bottom that may be capable of supporting the weight of the suppository when laid upon a flat surface.

A method of lubricating an internal surface of a body cavity may comprise the steps of providing a providing a suppository comprising a shell defining an internal volume and a lubricant occupying at least a portion of the internal volume, inserting the suppository into the body cavity, and allowing the shell to degrade inside the body cavity to release the lubricant. Insertion into the body cavity can be done by a user or medical technician via their hands, positioning the suppository and user (such as sitting on the suppository at the proper orientation), or the use of a tool operative to receive and insert the suppository. The body cavity could be an anus, vagina, or other anatomical body cavity of a human or non-human animal. The method could further include a step of performing a medical procedure associated with the body cavity; in such methods, the medical procedure could occur before, during, and/or after insertion of a suppository.

One or more suppositories can be included in a kit of parts for lubricating an internal surface of a body cavity. Each of the one or more suppositories can be packaged, in a wrapper for instance, to shield the respective suppository from an external environment. The kit could include other features, such as gloves, sterilization materials (e.g., spray, wipes), instructions, and/or a tool for inserting the suppository.

All of these embodiments are contemplated to be within the scope of this disclosure. These and other embodiments will become readily apparent to those skilled in the art form the following detailed description of the preferred embodiments having reference to the attached figures, the disclosure not being limited to any particular preferred embodiment.

### BRIEF DESCRIPTION OF THE DRAWINGS

These and other features and advantages of the various embodiments disclosed herein will be better understood with respect to the following description and drawings, in which like numbers refer to like parts throughout, and in which:
FIG. 1 is a front view of an exemplary embodiment of a suppository according to the present disclosure;
FIG. 2 is a bottom perspective view of an exemplary embodiment of a suppository according to the present disclosure;
FIG. 3 is a cross-sectional view of the suppository of FIGs. 1 and 2 along line 3-3 of FIG. 1;
FIG. 4 is the insertion of a suppository into a body cavity;
FIG. 5 is an internal view of a body cavity having a suppository recently inserted therein; and
FIG. 6 is the internal view of the body cavity of FIG. 5 with following degradation of the suppository shell.

Common reference numerals are used throughout the drawings and the detailed description to indicate the same elements.

### DETAILED DESCRIPTION

The present disclosure encompasses various embodiments of suppositories and methods of use thereof for delivering lubricant to an internal surface of a body cavity. A suppository may be composed of a lubricant and a shell surrounding and containing the lubricant. The suppository may have a smooth geometry which promotes insertion of the suppository into a body cavity. The shell of a suppository may be designed to degrade so that the lubricant may be released while inside the body cavity. A rate of degradation of the suppository's shell may be lower when packaged or external to the body cavity (i.e., a first condition) when compared to being exposed to an external environment or when inside of the body cavity (i.e., a second condition) due to, for example, moisture content in the air, heat absorbed by the suppository from the body cavity, or pressure applied to the suppository by the body cavity. Following partial or complete degradation of the suppository shell, lubricant may become applied to an internal surface of the body cavity, allowing for an activity such as a medical procedure (e.g., surgical or treatment-based) or a pleasure-based activity to be performed on the body cavity with aid from the lubricant.

The subject matter disclosed herein will be best understood in view of the drawings, in which exemplary embodiments of the systems and methods disclosed herein are shown. The drawings are intended to illustrate examples and are thus not intended to limit the systems and methods they embody. The detailed description set forth below in connection with the appended drawings is intended as a description of several currently contemplated embodiments and is not intended to represent the only form in which the disclosed invention may be developed or utilized. This description sets forth the functions and features in connection with the illustrated embodiments. It is to be understood, however, that the same or equivalent functions may be accomplished by different embodiments that are also intended to be encompassed within the scope of the present disclosure. It is further understood that the use of relational terms such as first and second and the like are used solely to distinguish one from another entity without necessarily requiring or implying any actual such relationship or order between such entities.

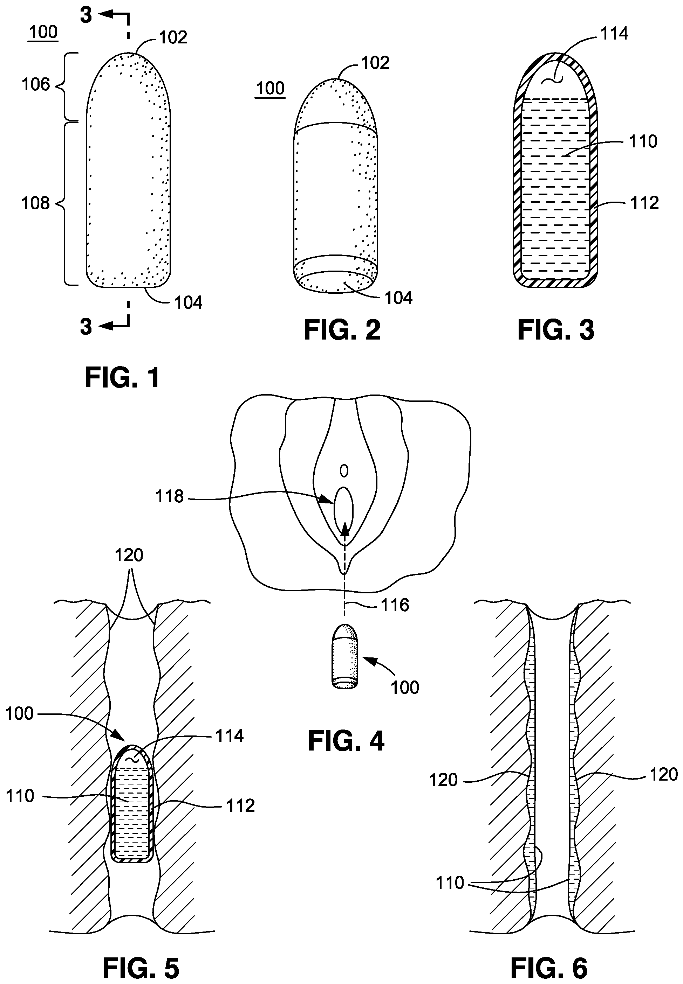

Referring first to FIGs. 1 and 2, a front view and a bottom perspective view respectively of an exemplary embodiment of a suppository according to the present disclosure are shown. A suppository 100 may have a smooth shape (with, for instance, conical or parabolic geometries) which promotes easy and comfortable insertion into a body cavity. As such, a suppository 100 would preferably have no sharp edges or rough surfaces which could inflict harm or irritation upon a user or patient receiving the suppository 100. A suppository 100 may be longer along a height thereof when compared to a width thereof so as to define a distal end 106 and a proximal end 108. The distal end 106 may gradually taper, ideally with a frustoconical geometry, until terminating at a tip 102. The body cavity may first receive the distal end 106, or specifically the tip 102, of the suppository 100 when inserting.

The proximal end 108 may define a maximum thickness of the suppository 100. In the case of a generally cylindrical proximal end 108, this maximum thickness may be a maximum diameter defined by the proximal end 108. A maximum thickness of a suppository 100 may be chosen based on the body cavity intended to receive the suppository 100, the anatomy of the user or patient receiving the suppository (and how that may correspond to an orifice size of the body cavity), and/or the amount of lubricant which one desires the suppository 100 to hold. The proximal end 108 of the suppository 100 may terminate at a bottom 104, which could be a smooth, flat surface on which the suppository 100 may stand upright when laid upon a flat surface like that of a table. The smooth bottom 104 may help to position the suppository 100 prior to insertion into a body cavity, as will be described later in this disclosure.

Turning now to FIG. 3, a cross-sectional view of the suppository of FIGs. 1 and 2 along line 3-3 of FIG. 1 is shown. A suppository 100 may be composed of a lubricant 110 and a shell 112. The shell 112 may define an outer surface and overall geometry of the suppository 100 and further define an internal volume which the lubricant 110 may occupy. While the opposite could be true in certain embodiments, this internal volume may not be entirely filled with lubricant 110, as a void 114 may occupy a portion of that internal volume. This void 114 could correspond to a gas trapped inside of the shell 112 (e.g., air, pure nitrogen) which could intentionally be present; alternatively, there could be no gasses trapped inside of the shell 112 and the void 114 could simply correspond to an internal volume of the shell 112 which lubricant 110 does not occupy. A suppository 100 could be primarily intended to deliver lubricant 110 to a body cavity, as opposed to, for example, the delivery of a therapeutic, and in this respect, a suppository could consist essentially of a shell 112 and a lubricant 110.

Suitable lubricants 110 include biocompatible lubricants known by those of ordinary skill in the art, such as those conventionally applied to the anus or vagina; specific examples include water-based lubricants, silicone-based lubricants, and oil-based lubricants (e.g., plant-based oils), although any biosafe lubricant known by those skilled in the art or those which are developed in the future can be used in the suppositories 100 disclosed herein. In certain embodiments, a lubricant 110 could comprise at least 20% by weight, at least 40% by weight, at least 60% by weight, or at least 80% by weight of the total weight of the suppository 100.

The shell 112 may be biocompatible, like the lubricant 110, and may have a lower rate of degradation in a first condition (e.g., when transporting or handling suppository 100) relative to the rate of degradation in a second condition (e.g., when the suppository 100 is inserted into a body cavity). The rate of degradation in the first condition may be minimal or zero, so long as a there is an appreciable increase in the second condition so that the shell 112 can degrade in the body cavity at a convenient time-scale. Degradation mechanisms the shell 112 may exhibit will be discussed later in this present disclosure. Particular materials the shell 112 may be made out of include polymers like agarose, fibrin, fibrinogen, collagen, alginate, chitosan, methylcellulose, polyglycolide, poly(lactide-co-glycolide) acid (or PLGA), polyethylene glycol, and combinations thereof. In addition, any material known by those skilled in the art or a future developed material which is found to be biosafe when degraded within the body cavity which the suppository 100 is intended to be inserted into can be used a material of the shell 112.

Referring now to FIG. 4, the insertion of a suppository into a body cavity is depicted. A suppository 100 can be inserted 116 into a body cavity 118, which could be a human user's anus, vagina, or other suitable anatomical orifice. However, the body cavity 118 could also belong to a non-human animal if, for instance, a medical procedure is to be performed related to that animal's body cavity. Prior to insertion, a suppository 100 could be packaged to prevent premature degradation of the shell 112 and inadvertent release of the lubricant 100; if the shell 112 is made of a material which is prone to degradation when exposed to ambient moisture content from an external environment, for example, this may be necessary if the suppository 100 is to be transported/stored prior to insertion 116 into a body cavity 118. The package could come in the form of a wrapper which could be torn open to expose the suppository 100 to an external environment.

A suppository 100 could be inserted 116 into a body cavity 118 through a number of methodologies. Manual insertion of the suppository 100 could involve a user using their hands to insert the suppository 100 into their own body cavity 118 or a medical technician using their hands to insert the suppository 100 into a patient's body cavity 118. A proximal end 108 of a suppository 100 may be configured to promote the handling of a suppository 100 for this purpose, such as smooth indentations that can receive one's fingers. If the bottom 104 of a suppository 100 can support the weight of the suppository 100 and allow it to stand upright when laid upon a flat surface, a user or technician could position the suppository so that the user/a patient can sit or squat upon the suppository 100 to insert 116 it into the body cavity 118. One could also use a tool to insert a suppository 100; this tool could be a hand-held or larger instrument which a user or medical technician could operate so slowly insert the suppository into the body cavity 118 when the tool and user/patient are properly positioned. The tool could rely on any mechanical process for transporting the suppository 100, including, but not being limited to, piston or air-pressure activation. Using a tool may be preferable when inserting a suppository 100 deeper into a body cavity 118, as alternative mechanisms may have a limited insertion range. Whichever insertion mechanism is used, the suppository 100 would preferably be inserted such that the body cavity first receives a distal end 106 of the suppository 100.

A suppository 100 may be packaged as a kit of parts, including any of the following: one or more suppositories (each of which could be individually packaged in an airtight package), one or more pairs of gloves, an insertion tool, sterilization materials (e.g., spray, wipes), and instructions. These kits of parts could be sold as products for user or patients to treat themselves or to medical technicians for treating their patients.

Looking now to FIG. 5, an internal view of a body cavity having a suppository recently inserted therein is portrayed. Upon insertion into a body cavity, a suppository 100 may be adjacent to internal walls 120 of the body cavity. Initially, the shell 112 of the suppository 100 may be structurally stable while inside the body cavity, thus containing the lubricant 110 contained therein. Depending on the configuration of the suppository 100, a rate of degradation of the shell 112 may increase prior to insertion into the body cavity. This could be the case if the shell 112 is composed of a material which weakens when exposed to an external environment (e.g., moisture content in the air). This type of suppository 100 may, soon after manufacture thereof, be sealed in an airtight package with low moisture content. When one desires to insert this suppository into a body cavity, they can open the package and insert the suppository before significant degradation of the shell 112 occurs. The rate of degradation of these types of suppositories may increase even further following insertion thereof into the body cavity, which could a third condition.

Preferably, however, the shell 112 would maintain a low rate of degradation when exposed prior to insertion of the suppository 100 into the body cavity and have a significant increase in its rate of degradation after insertion. This could be a result of the shell 112 being composed of a material which weakens when absorbing heat from the body cavity and/or weakens from pressure exerted on the suppository by the internal walls 120. With this arrangement, the suppository 100 could be transported and stored with greater ease and the degradation process can be made more predictable and controllable.

Referring now to FIG. 6, the internal view of the body cavity of FIG. 5 following degradation of the suppository shell is shown. With the shell degraded, the lubricant 110 originally contained therein is released and applied to the internal walls 120 of the body cavity. The thickness and shape of the suppository may result in more lubricant 110 being applied to certain internal surfaces of the body cavity compared to others. While a suppository which is generally symmetrical along a proximal-to-distal direction may result in the application of lubricant evenly upon the body cavity's internal surfaces, another suppository whose shell is thicker in certain regions may take a longer time to degrade when compared to thinner regions of the shell. As such, most, if not all, of the lubricant 110 may be released through an opening formed during an initial degradation of the suppository shell, with the opening's position on the suppository corresponding to the thinner regions of the original shell. If one wished to apply the lubricant 110 to a certain area of the internal surfaces 120 in this manner, it may require that the suppository be inserted into the body cavity at a particular orientation such that the thinner regions of the shell are adjacent to the target area of the internal surfaces 120. It can be appreciated that by virtue of the suppository being inserted at a certain depth into the body cavity and the lubricant 110 spreading from that depth, lubricant 110 can cover areas of the internal surfaces which would otherwise be challenging or inconvenient to reach with prior lubrication products and methodologies.

With the lubricant 110 now applied to the internal surfaces 120, one can proceed with any activities or operations which may rely upon or be aided in part by the internal surfaces 120 being lubricated. Alternatively, or additionally, suppositories 100 can be inserted in the middle of or after such activities. These activities can include performing any medical operations associated with the body cavity or pleasurable activities, like using a sex toys, which could be associated with the body cavity. In this respect, the devices and methodologies disclosed in Applicant's previously filed patent applications may be employed. These include US Patent Publication No. 2023/0201071 entitled "ENHANCED PROPHYLACTICS FOR VACUUM THERAPY", US Patent Publication No. 2023/0293863 entitled "IMPROVED DILATION DEVICE INCORPORATING INFLATABLE BALOON", US Patent Publication No. 2023/0414448 entitled "DILATION DEVICE INCORPORATING ENHANCED FEATURES", US Patent Publication No. 2024/0082552 entitled "RESILIENT ADHESIVE SHEATH FOR USE WITH DILATION DEVICE", US Patent Application No. 18/186,820 entitled "SYSTEMS AND METHODS FOR IMPROVED ENHANCED DILATION DEVICES FOR TARGETED DILATION DIAGNOSTSICS AND THERAPIES", and US Patent Application No. 18/297,428 entitled "SYSTEM AND METHOD FOR TREATING FECAL IMPACTION", the disclosures of each of which are hereby wholly incorporated by reference.

The above description is given by way of example, and not limitation. Given the above disclosure, one skilled in the art could devise variations that are within the scope and spirit of this disclosure. Further, the various features of the embodiments disclosed herein can be used alone, or in varying combinations with each other and are not intended to be limited to the specific combination described herein. Thus, the scope of the claims is not to be limited by the illustrated embodiments. Additional modifications and improvements of the present disclosure may also be apparent to those of ordinary skill in the art. Thus, the particular combination of parts and steps described and illustrated herein is intended to represent only certain embodiments of the present subject matter and is not intended to serve as limitations of alternative devices and methods within the spirit and scope of this disclosure.
The following embodiments shall also be considered as disclosed:
Embodiment 1:
   A method for lubricating an internal surface of a body cavity, the method comprising the steps of: providing a suppository comprising: a shell defining an internal volume; and a lubricant occupying at least a portion of the internal volume; inserting the suppository into the body cavity; and allowing the shell to degrade inside the body cavity to release the lubricant; wherein a rate of degradation of the shell increases after insertion of the suppository into the body cavity.
Embodiment 2:
   The method of embodiment 1, wherein the lubricant is at least 20% by weight of a total weight of the suppository
Embodiment 3:
   The method of embodiment 1, wherein said step of inserting the suppository comprises using of a tool operative to receive the suppository to insert the suppository into the body cavity.
Embodiment 4:
   The method of embodiment 1, wherein the lubricant is a water-based lubricant, a silicone-based lubricant, or an oil-based lubricant.
Embodiment 5:
   The method of embodiment 1, wherein the shell comprises agarose, fibrin, fibrinogen, collagen, alginate, chitosan, methylcellulose, polyglycolide, poly(lactide-co-glycolide) acid, polyethylene glycol, or combinations thereof.
Embodiment 6:
   The method of embodiment 1, wherein the body cavity is an anus or a vagina.
Embodiment 7:
   The method of embodiment 1, wherein the increase in the rate of degradation of the suppository is a result of at least: the suppository absorbing heat from the body cavity, the body cavity applying pressure to the suppository, or both.
Embodiment 8:
   The method of embodiment 1, wherein the method further comprises a step of: performing a medical procedure associated with the body cavity.
Embodiment 9:
   The method of embodiment 1, wherein the suppository has a proximal end and a distal end, the distal end comprising a tip.
Embodiment 10:
   A suppository for lubricating an internal surface of a body cavity, the suppository consisting essentially of: a shell defining an internal volume; and a lubricant occupying at least a portion of the internal volume; wherein a rate of degradation of the shell increases after insertion of the suppository into the body cavity.
Embodiment 11:
   The suppository of embodiment 10, wherein the lubricant is at least 20% by weight of a total weight of the suppository.
Embodiment 12:
   The suppository of embodiment 10, wherein the lubricant is a water-based lubricant, a silicone-based lubricant, or an oil-based lubricant.
Embodiment 13:
   The suppository of embodiment 10, wherein the shell comprises agarose, fibrin, fibrinogen, collagen, alginate, chitosan, methylcellulose, polyglycolide, poly(lactide-co-glycolide) acid (or PLGA), polyethylene glycol, or combinations thereof.
Embodiment 14:
   The suppository of embodiment 10, wherein the increase of the rate of degradation following insertion into the body cavity is a result of at least: heat absorbed by the suppository from the body cavity, pressure applied to the suppository by the body cavity, or both.
Embodiment 15:
   The suppository of embodiment 10, wherein the suppository has a proximal end and a distal end, the distal end comprising a tip.
Embodiment 16:
   A kit for lubricating an internal surface of a body cavity comprising: one or suppositories, each of the suppositories individually consisting essentially of: a shell defining an internal volume; and a lubricant occupying at least a portion of the internal volume; wherein each of the suppositories are individually contained in a package shielding the respective suppositories from an external environment; and wherein a rate of degradation of the shell increases when the suppositories are exposed to the external environment.
Embodiment 17:
   The kit of embodiment 16, wherein the lubricant of each of the one or more suppositories is at least 20% by weight of a total weight of the respective suppository.
Embodiment 18:
   The kit of embodiment 16, wherein the lubricant of each of the one or more suppositories are independently a water-based lubricant, a silicone-based lubricant, or an oil-based lubricant.
Embodiment 19:
   The kit of embodiment 16, wherein the shell of each of the one or more suppositories independently comprises agarose, fibrin, fibrinogen, collagen, alginate, chitosan, methylcellulose, polyglycolide, poly(lactide-co-glycolide) acid (or PLGA), polyethylene glycol, or combinations thereof.
Embodiment 20:
   The kit of embodiment 16, wherein the rate increase in the rate of degradation of the shell is a result of at least: an exposure to an increase moisture content following release of the suppositories from their respective packages.

## Claims

1. A method for lubricating an internal surface of a body cavity, the method comprising the steps of:
providing a suppository comprising:
a shell defining an internal volume; and
a lubricant occupying at least a portion of the internal volume;
inserting the suppository into the body cavity; and
allowing the shell to degrade inside the body cavity to release the lubricant;
wherein a rate of degradation of the shell increases after insertion of the suppository into the body cavity.

2. The method of claim 1,
wherein the lubricant is at least 20% by weight of a total weight of the suppository, and/or
wherein said step of inserting the suppository comprises using of a tool operative to receive the suppository to insert the suppository into the body cavity.

3. The method of claim 1,
wherein the lubricant is a water-based lubricant, a silicone-based lubricant, or an oil-based lubricant, and/or
wherein the shell comprises agarose, fibrin, fibrinogen, collagen, alginate, chitosan, methylcellulose, polyglycolide, poly(lactide-co-glycolide) acid, polyethylene glycol, or combinations thereof.

4. The method of claim 1,
wherein the body cavity is an anus or a vagina, and/or
wherein the increase in the rate of degradation of the suppository is a result of at least: the suppository absorbing heat from the body cavity, the body cavity applying pressure to the suppository, or both.

5. The method of claim 1, wherein the method further comprises a step of:
performing a medical procedure associated with the body cavity.

6. The method of claim 1, wherein the suppository has a proximal end and a distal end, the distal end comprising a tip.

7. A suppository for lubricating an internal surface of a body cavity, the suppository consisting essentially of:
a shell defining an internal volume; and
a lubricant occupying at least a portion of the internal volume;
wherein a rate of degradation of the shell increases after insertion of the suppository into the body cavity.

8. The suppository of claim 7,
wherein the lubricant is at least 20% by weight of a total weight of the suppository, and/or
wherein the lubricant is a water-based lubricant, a silicone-based lubricant, or an oil-based lubricant.

9. The suppository of claim 7,
wherein the shell comprises agarose, fibrin, fibrinogen, collagen, alginate, chitosan, methylcellulose, polyglycolide, poly(lactide-co-glycolide) acid (or PLGA), polyethylene glycol, or combinations thereof, and/or
wherein the increase of the rate of degradation following insertion into the body cavity is a result of at least: heat absorbed by the suppository from the body cavity, pressure applied to the suppository by the body cavity, or both.

10. The suppository of claim 7, wherein the suppository has a proximal end and a distal end, the distal end comprising a tip.

11. A kit for lubricating an internal surface of a body cavity comprising:
one or suppositories, each of the suppositories individually consisting essentially of:
a shell defining an internal volume; and
a lubricant occupying at least a portion of the internal volume;
wherein each of the suppositories are individually contained in a package shielding the respective suppositories from an external environment; and
wherein a rate of degradation of the shell increases when the suppositories are exposed to the external environment.

12. The kit of claim 11, wherein the lubricant of each of the one or more suppositories is at least 20% by weight of a total weight of the respective suppository.

13. The kit of claim 11, wherein the lubricant of each of the one or more suppositories are independently a water-based lubricant, a silicone-based lubricant, or an oil-based lubricant.

14. The kit of claim 11, wherein the shell of each of the one or more suppositories independently comprises agarose, fibrin, fibrinogen, collagen, alginate, chitosan, methylcellulose, polyglycolide, poly(lactide-co-glycolide) acid (or PLGA), polyethylene glycol, or combinations thereof.

15. The kit of claim 11, wherein the rate increase in the rate of degradation of the shell is a result of at least: an exposure to an increase moisture content following release of the suppositories from their respective packages.
